# EUROPEAN PATENT APPLICATION

(11) **EP 1 574 585 A1**
(43) Date of publication of application: **14.09.2005**
(21) Application number: 04075839.3
(22) Date of filing: 12.03.2004
(51) Int. Cl.: C12Q 1/68

(54) **Method for selective amplification of DNA fragments for genetic fingerprinting**

(71) Applicant: Plant Research International B.V., 6708 PB Wageningen (NL)
(72) Inventor: Schouten, Henk J., c/o Plant Research Int. B.V., 6708 PB Wageningen (NL); Van der Linden, C. Gerard, Plant Res. Int. B.V., 6708 PB Wageningen (NL)
(74) Representative: De Hoop, Eric

(57) **Abstract**

The invention discloses a method for preparing a reproducible selective set of DNA fragments from the DNA or cDNA of one or more organisms as a main step in a DNA fingerprinting method. The method comprises
a) cutting DNA with one or more restriction enzymes to obtain a set of double stranded DNA fragments,
b) ligating to the DNA fragments double stranded adapters that match the generated fragment ends, and
c) performing a polymerase chain reaction using primers which are specific for the adapters to obtain the reproducible selective set of DNA fragments.

In step b) at least one modified adapter is used which is composed of two oligonucleotides that are only partly complementary, leaving at least one part of the adapter single stranded, in such a way that a complementary strand of said single stranded part of the adapter cannot be synthesized from an available 3'-end in the adapter itself, and in step c) the primer which is specific for the modified adapter, is identical or similar to the single stranded part of the adapter, such that only after complementation of the single stranded part of the adapter said primer can anneal to the complement, and in such a way that this primer can be extended in the direction of the ligation site.

SEQUENCE LISTING

<110> Plant Research International B.V.

<120> Method for selective amplification of DNA fragments for
      genetic fingerprinting

<130> EPP172097

<140> EP 04075839.3
<141> 2004-03-12

<160> 16

<170> PatentIn Ver. 2.1

<210> 1
<211> 32
<212> DNA
<213> Artificial Sequence

<220>
<223> Description of Artificial Sequence: adapter long
      strand

<400> 1
actcgattct caacccgaaa gtatagatcc ca                                32

<210> 2
<211> 14
<212> DNA
<213> Artificial Sequence

<220>
<223> Description of Artificial Sequence: adapter short
      strand

<400> 2
tgggatctat actt                                                    14

<210> 3
<211> 25
<212> DNA
<213> Artificial Sequence

<220>

<223> Description of Artificial Sequence: NBS-specific
      primer

<220>
<221> misc_feature
<222> (11)..(12)
<223> modified base i

<220>
<221> misc_feature
<222> (22)..(23)
<223> modified base i

<400> 3
yytkrthgtm ntkgatgatg tntgg                                          25


<210> 4
<211> 21
<212> DNA
<213> Artificial Sequence

<220>
<223> Description of Artificial Sequence: PASCAS primer

<400> 4
actcgattct caacccgaaa g                                              21


<210> 5
<211> 21
<212> DNA
<213> Artificial Sequence

<220>
<223> Description of Artificial Sequence: adapter strand

<400> 5
ctcgtagact gcgtacatgc a                                              21


<210> 6
<211> 14
<212> DNA
<213> Artificial Sequence

<220>
<223> Description of Artificial Sequence: complementary

adapter strand

<400> 6
tgtacgcagt ctac                                                    14

<210> 7
<211> 19
<212> DNA
<213> Artificial Sequence

<220>
<223> Description of Artificial Sequence: primer

<400> 7
gtagactgcg tacatgcag                                               19

<210> 8
<211> 32
<212> DNA
<213> Artificial Sequence

<220>
<223> Description of Artificial Sequence: PASCAS adapter
      strand

<400> 8
actcgtatct caacccgaaa gtatagactc ca                                32

<210> 9
<211> 19
<212> DNA
<213> Artificial Sequence

<220>
<223> Description of Artificial Sequence: complementary
      PASCAS adapter strand with block

<400> 9
aatttggagt ctatacttm                                               19

<210> 10
<211> 18
<212> DNA
<213> Artificial Sequence

<220>
<223> Description of Artificial Sequence: primer

<400> 10
actcgtatct caacccga                                                    18


<210> 11
<211> 21
<212> DNA
<213> Artificial Sequence

<220>
<223> Description of Artificial Sequence: adapter strand

<400> 11
gtgctacagt cgctagatgc a                                                21


<210> 12
<211> 14
<212> DNA
<213> Artificial Sequence

<220>
<223> Description of Artificial Sequence: complementary
      adapter strand

<400> 12
tctagcgact gtag                                                        14


<210> 13
<211> 16
<212> DNA
<213> Artificial Sequence

<220>
<223> Description of Artificial Sequence: primer

<400> 13
cagtcgctag atgcag                                                      16


<210> 14
<211> 32
<212> DNA

<213> Artificial Sequence

<220>
<223> Description of Artificial Sequence: PASCAS adapter
      strand

<400> 14
agtgctatgt gaagggcaaa ctatacagtg ga                                32

<210> 15
<211> 19
<212> DNA
<213> Artificial Sequence

<220>
<223> Description of Artificial Sequence: complementary
      PASCAS adapter strand with block

<400> 15
aatttccact gtatagttm                                               19

<210> 16
<211> 18
<212> DNA
<213> Artificial Sequence

<220>
<223> Description of Artificial Sequence: primer

<400> 16
agtgctatgt gaagggca                                                18

## Description

This application relates to a method for the selective amplification of particular DNA fragments during a polymerase chain reaction (PCR) for genetic fingerprinting techniques.

### Background of the invention

Genotyping is the process of describing the genetic constitution of an organism or cell. DNA fingerprinting techniques are the genotyping tools that can reveal differences and similarities between the genetic material of individuals. These differences between related organisms can be used as genetic marker. DNA fingerprinting is important for commercial breeding programs, identification of individual organisms for e.g. diagnostics and monitoring, determination of parentage, investigation of inheritance of genetic traits, and so on. A wide range of DNA fingerprinting methods exists, including Random Amplified Polymorphic DNA (RAPD), AFLP®, Simple Sequence Repeats (SSRs), and Diversity Array Technology (DArT).

DNA fingerprinting methods that target multiple loci (like for instance RAPD, AFLP, and DArT) usually comprise as a main step the reproducible creation of a selective set of DNA fragments from the genomic DNA of one or more organisms. This is called a diversity panel.
A variety of methods for making diversity panels exist, some of which are described in WO 01/73119 . Some methods for creation of diversity panels, all based on the polymerase chain reaction (PCR) and most of these used in DNA fingerprinting techniques, are the following:
RAPD (Random Amplified Polymorphic DNA) is a molecular marker method that uses short arbitrary oligonucleotides for random amplification of genomic DNA fragments (Williams *et al*., 1990). With RAPD, DNA fragments containing two primer binding sites in opposite orientation (pointing towards each other) and close enough to allow PCR amplification will be amplified and present in the diversity panel, thus achieving complexity reduction. Depending on the genome size and composition, the number of fragments can be varied with the length of the primers (generally 8-10 nucleotides). While simple to perform, fast and relatively cheap, RAPD assays often suffer from limited reproducibility. This is most likely related to the use of short primers in a PCR reaction at low stringency annealing temperatures for generation of the diversity panel.
A method for reduction of the number of amplified fragments is applied in the genotyping method AFLP (Vos *et al*., 1995). In AFLP, DNA is digested with restriction enzymes, double-stranded adapters are ligated to the fragments, and fragments are amplified with PCR using primers that anneal to the adapters. The amplification primers in AFLP are extended beyond the sequence of the adapter and restriction site with one or more selective bases. When the primer has one extra base, the frequency of sites where this primer can anneal perfectly is reduced to approximately ¼ = 25 %. This reduces the number of amplified fragments. Using more selective bases further reduces the number of fragments in the diversity panel and therefore the complexity of the diversity panel.
Another method for diversity panel generation is described by Jaccoud *et al*. (2001). They used this method for the fingerprinting technique DArT (Jaccoud *et al*., 2001; patent application WO 01/73119). The method starts by cutting the DNA with a restriction enzyme. This gives rise to a set of double stranded DNA fragments. This set is in most cases too large and too complex to be used for the fingerprinting method DArT. To reduce the complexity, a selective set of fragments is amplified by adapter-mediated amplification. For this, a double-stranded adapter is attached to both ends of all the fragments. Then PCR is performed, using a primer that anneals to the adapter. The created set of amplicons is the diversity panel. The complexity reduction arises from a PCR limitation itself: very long fragments are usually not amplified efficiently. This technical limitation of the PCR provides a filter, giving a preferred amplification of shorter fragments. This reduces the complexity of the set of amplified fragments. However, the lower size threshold for fragments that will be amplified in PCR is not clear-cut and depends on many factors. Therefore the fragment composition of this diversity panel may vary, which will affect reproducibility of genotyping.
Chenchik *et al*. (patent application WO96/23079) describe a method to suppress amplification of particular DNA fragments during PCR. They also use adapters that are ligated to the ends of DNA restriction fragments. A single stranded DNA fragment has self-complementary adapters at the 5'- and 3'-end. These two ends may anneal to one another, thus forming a "pan-like" double stranded structure. Such a "pan-like" structure reduces primer annealing to the adapters. As a result, amplification of these fragments is suppressed. Although this method was not primarily meant for complexity reduction for fingerprinting, it offers interesting possibilities for it. A limitation may be that it suppresses rather than completely excludes amplification of particular DNA fragments. In addition, especially for long fragments PCR suppression is not very efficient.

In other embodiments DNA elements with identical sequences that are present in many copies scattered over the genome are utilised for selective amplification. Usually one primer anneals to a sequence in the DNA element, whereas another primer anneals to a sequence flanking the element. Kilian describes in his patent application (WO 01/73119) an example of using transposon insertions in rice for making diversity panels for DArT. The primer that anneals to an element is preferably designed in such a way that it anneals to an edge of the element, the 3' end pointing outward. For amplification of the flanking sequences the DNA is cut with a restriction enzyme, adapters are attached to the fragment ends, and an adapter-specific primer is used together with the element-specific primer in PCR. A similar approach of using retrotransposon elements in combination with steps from the AFLP protocol was used for gel-based genotyping by Waugh et al (1997). A limitation of using repeated elements such as transposons is that the primer design requires sequence information of the element. This information is often not directly available. Further, the number of elements in the genome, and therewith the complexity of the diversity panel, varies between crops. For each crop a suitable target element needs to be identified.

Similarly, conserved sequences may be used that are present in many copies in a genome, such as in gene families like the resistance gene family containing Nucleotide Binding Site-Leucine Rich Repeats (NBS-LRR), MADS box genes, protein kinases, and so on. Examples of genotyping methods using primers directed to gene family sequences are motif-directed approaches like the modified AFLP approach with NBS-LRR gene-specific primers as reported by Hayes and Saghai Maroof (2000), and ligation-mediated PCR (LM-PCR) (Hornstra and Yang, 1993).

It is also possible to use a simple sequence repeat as an element that is present at multiple loci in a genome (WO 01/088189 A3). The primer that anneals to the simple sequence repeat is then preferably designed in such a way that it contains complementary sequences of a part of the repeat, and one or a few additional bases at the 3' end. By doing so, the primer is anchored at the 3' end of the simple sequence repeat. Like in the approach for the repeated elements, the other primer should anneal to a flanking sequence.

### Summary of the invention

The present invention describes an approach for generating diversity panels that provides a significant improvement in the application of these panels for several fingerprinting techniques.

A method for creation of diversity panels for fingerprinting techniques should be highly reproducible. Further, it should allow adjustment of the number of fragments for obtaining an optimal complexity of the diversity panel for each target organism. Moreover, the percentage of fragments that do reveal genetic differences between closely related organisms should be as high as possible.

The present invention provides a method for preparing a reproducible selective set of DNA fragments from the DNA or cDNA of one ore more organisms as a main step in a DNA fingerprinting method, comprising
a) cutting DNA with one or more restriction enzymes to obtain a set of double stranded DNA fragments,
b) ligating to the DNA fragments double stranded adapters that match the generated fragment ends, and
c) performing a polymerase chain reaction using primers which are specific for the adapters to obtain the reproducible selective set of DNA fragments,
characterized in that in step b) at least one modified adapter is used which is composed of two oligonucleotides that are only partly complementary, leaving at least one part of the adapter single stranded, in such a way that a complementary strand of said single stranded part of the adapter cannot be synthesized from an available 3'-end in the adapter itself, and in step c) the primer which is specific for the modified adapter, is identical or similar to the single stranded part of the adapter, such that only after complementation of the single stranded part of the adapter said primer can anneal to the complement, and in such a way that this primer can be extended in the direction of the ligation site.

The DNA starting material which is cut (digested) in step a) can be genomic DNA, mitochondrial DNA, chloroplast DNA or cDNA from any organism. In the method of the invention the adapter is composed of two oligonucleotides that are only partly complementary, leaving at least one part of the adapter single stranded. One oligonucleotide is protruding at its 5'- end and is not complemented for generally at least 8 nucleotides, preferably 15 - 30 nucleotides. The accompanying amplification primer is identical or similar to this single stranded part of the adapter. Consequently, the primer cannot anneal to the adapter. However, if the single stranded part of the adapter is complemented, the primer can anneal to this complement. This implies that the PCR amplification can not be initiated from this adapter and the accompanying amplification primer. First, the complementary sequence of the single stranded part of the adapter should be synthesized. Then the complement of the single stranded part can serve as a template for the accompanying amplification primer. The adapter is modified in such a way that the synthesis of a complementary strand to which the adapter primer can anneal can not initiate from an available 3'-end in the adapter itself. Synthesis of the complement of the single stranded part of the modified adapter requires initiation from a priming site outside the modified adapter.

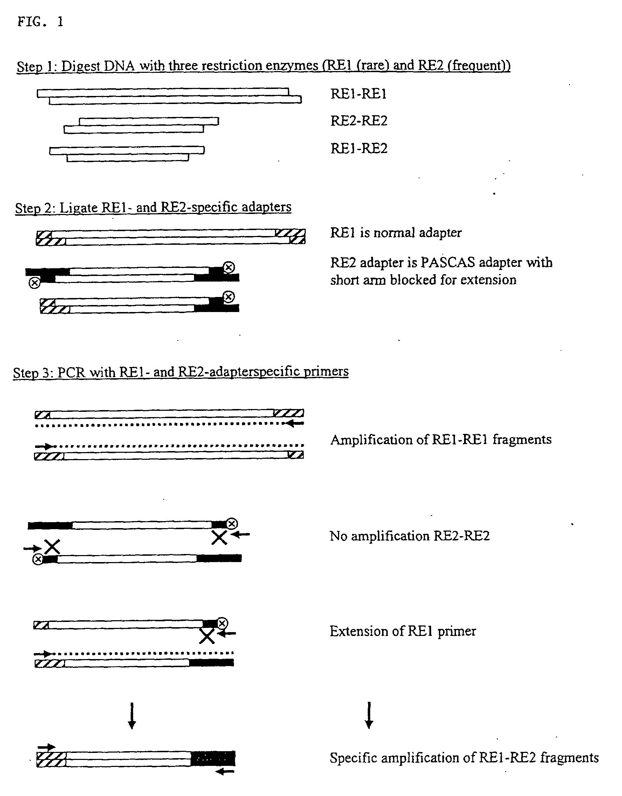

This kind of primer-adapter combination we have named Primer Annealing Site after Complementation of Adapter Strand (PASCAS). If a restriction fragment has at both ends a PASCAS adapter, and if there is no annealing site for the PASCAS primer or other present primers in the fragment itself, then this fragment is not amplified. However, if a restriction fragment has e.g. at the one end a normal adapter and at the other end a PASCAS adapter, then the accompanying primer for the normal adapter can anneal this adapter. In this context the term "normal adapter" means a double-stranded adapter which already contains an annealing site for the accompanying PCR primer and therefore allows primer extension without prior synthesis of said primer annealing site. Elongation of the primer may lead to complementation of the restriction fragment and of one PASCAS adapter strand, therewith providing a template for the PASCAS primer. Then, PCR amplification can start. This is illustrated in Fig. 1.

A PASCAS adapter and accompanying primer provide a means for a reproducible creation of a diversity panel.

Examples of PASCAS adapters are the blocked adapter, the vectorette adapter and the spinklerette adapter. These adapters have been described earlier (Hui *et al*., 1998). The blocked adapter is described in the user manual of the Universal GenomeWalker™ Kit of CLONTECH Laboratories (PT3042-1, page 29). This kit from CLONTECH applies to the blocked adapter in combination with PCR suppression. The vectorette adapter is referred to in numerous papers on isolating and sequencing flanking regions of known DNA sequences. These adapters were not used for creation of diversity panels for fingerprinting techniques, but for isolating and sequencing DNA flanking known sequences.

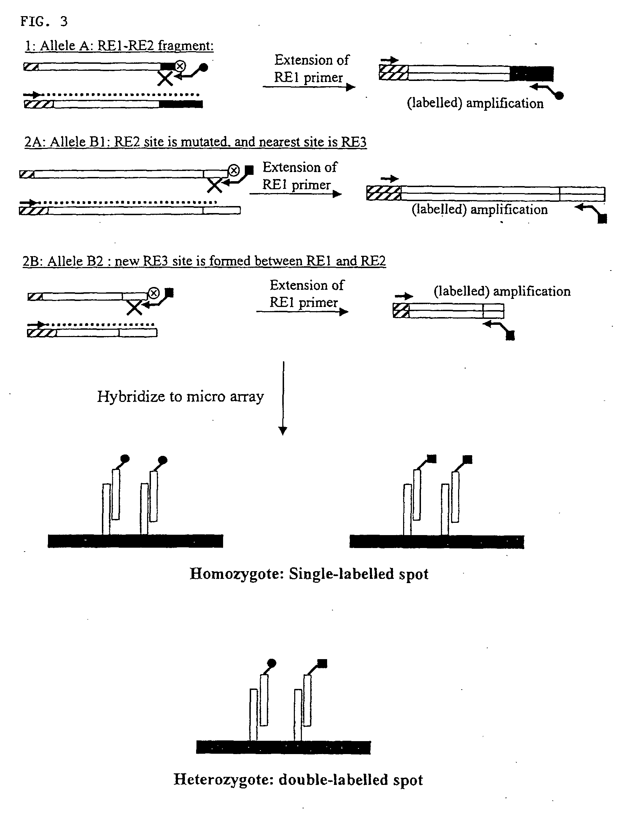

The use of the PASCAS system for generation of diversity panels has specific advantages:
- The generation of diversity panels with a PASCAS system is highly reproducible.
   Other common methods for reduction of the complexity of a diversity panel are less reproducible.
   Reduction of the complexity by excluding long fragments because of the reduced ability of polymerase to amplify long fragments does not provide a clear-cut selection. The maximum fragment size for which amplification still occurs is not clear-cut, and depends on several conditions.
   Another common reduction method is the use of selective bases, such as for AFLP. However, one mismatch at the 3' end of an adapter primer usually does not completely prevent amplification, but reduces the amplification efficiency.
   As a result, also the reduction in complexity by means of selective bases is not clear-cut, and depends on annealing conditions.
- Different PASCAS adapters can be used simultaneously in a single PCR. This flexibility offers extra possibilities for generation of diversity panels and genotyping. Figure 3 illustrates the use of two different PASCAS adapters for codominant scoring. Codominant scoring provides not only information on presence of one allele, but also on presence of other allele(s), and therewith on heterozygosity. Codominant scoring is usually preferred to dominant scoring, which provides information on presence of one allele only.

### Description of the drawings

In Figures 1 - 4 various embodiments of the method of the invention are shown.

Figure 1 schematically describes the use of the PASCAS adapter system to create a diversity panel by selectively blocking amplification. The genomic DNA is digested with two restriction enzymes (RE1 and RE2), with RE1 preferably a rare-cutting enzyme (recognizing a restriction enzyme site of for example six base pairs or more) and RE2 a frequent cutter (for example, recognizing four base pairs). This generates three types of fragments: fragments cut by RE1 only (RE1-RE1), which will be relatively rare, fragments cut by only RE2 (RE2-RE2), which will be abundant, and RE1-RE2 fragments. In a second step, two types of adapters are ligated to the fragments: A RE1 adapter that can ligate to the RE1-ends of the fragments, and a PASCAS RE2 adapter that ligates to the RE2-ends of the fragments. Then PCR is performed with amplification primers for RE1 and RE2 adapters. The RE1 adapter primer can directly bind to the RE1 adapter. Therefore, the RE1-RE1 fragments will be amplified, but these will be rare. The RE2 adapter primer can not directly bind to the RE2 adapter. Therefore, RE2-RE2 fragments will not be amplified. However, in RE1-RE2 fragments a first strand is synthesized by extension of the RE1 primer. This creates an annealing site for the RE2 primer, allowing amplification of the RE1-RE2 fragments.

An optional step can be included to further decrease complexity. This step involves the use of a third restriction enzyme (RE3, preferably a frequent cutting enzyme). RE3 is used after step 2. Fragments that are cut with RE3 will not be amplified in PCR in step 3. This reduces the complexity of the diversity panel. Longer fragments are more likely to be cut by RE3 than short fragments. The remaining fragments are generally short enough for efficient amplification. In such a way, reduction of the complexity of the diversity panel by less efficient amplification of long fragments may be prevented. This optional step is not shown in Figure 1.

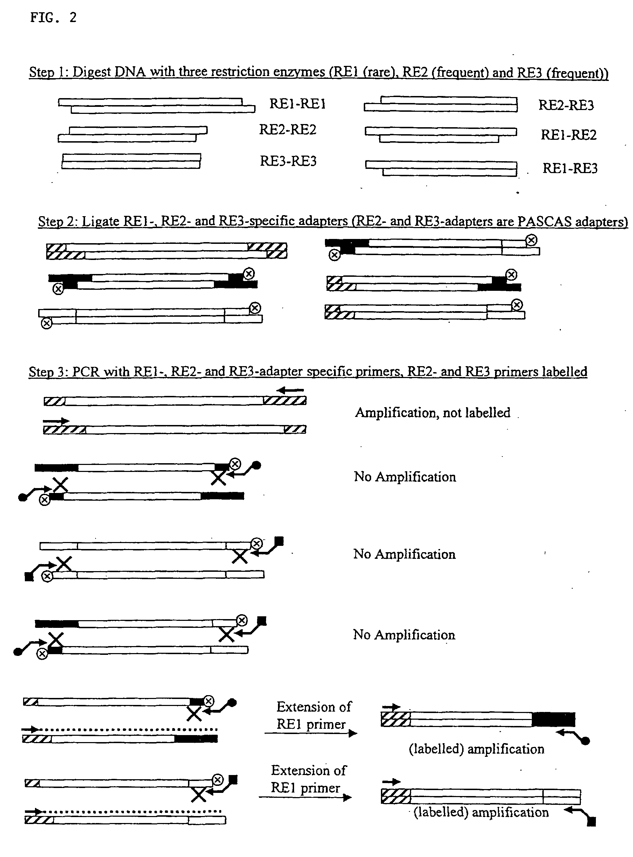

Figure 2 schematically describes the use of two PASCAS adapters to create a diversity panel, which allows for the detection of two sets of fragments separately. In step 1, genomic DNA is digested with three restriction enzymes (RE1, RE2 and RE3), with RE1 preferably a rare cutting restriction enzyme, and RE2 and RE3 preferably frequent cutting enzymes. In step 2, three RE-specific adapters are ligated to the fragments. The RE1 adapter is a normal adapter, RE2-and RE3 adapters are PASCAS adapters. Next, PCR is performed using primers for the three adapters. The PASCAS primers are labeled, preferably with labels that can be detected separately (for instance, two fluorescent labels with different colors). Only fragments with normal adapters at one or both ends will be amplified. These are RE1-RE1 fragments (which will be rare), RE1-RE2 and RE1-RE3 fragments. Because of the different labels for RE2 and RE3 PASCAS primers, the two sets of fragments can be detected separately.

Figure 3 schematically describes codominant scoring of markers by making use of two PASCAS adapters to create a diversity panel as described in Figure 2, and subsequent fingerprinting with DArT. Step 3 in Figure 2 describes the selective amplification of RE1-RE2 and RE1-RE3 fragments. Figure 3 describes the situation where a genetic (allelic) variation results in the conversion of an RE1-RE2 fragment into an RE1-RE3 fragment. In the case of 2A, the RE2-site is destroyed by genetic variation (insertions/deletions or substitutions or any other variation that inhibits the cutting of this site by RE2), and the next available restriction site (downstream of the disappeared RE2 site) is an RE3 site. The original RE1-RE2 fragment is essentially changed into an RE1-RE3 fragment (allele B1). As amplification primers for RE2 and RE3 site are differently labeled, the new allele B1 will have a different label compared to allele A. In Fig. 2B, the RE2 site is not destroyed. Instead, genetic variation has created an extra RE3 site within the RE1-RE2 fragment (allele B2). After amplification, allele B2 is labeled with the RE3 label. The different alleles (A vs B1/B2) can be visualized by hybridization to a diversity array. On the array, the original RE1-RE2 fragment is present in a hybridization spot. Both A and B alleles can hybridize to this fragment. In the case of a homozygote AA, only RE1-RE2 will hybridize, resulting in a RE2-label signal on this spot. In the case of a BB homozygote, only RE1-RE3 fragments will hybridize, resulting in a single RE3-label signal. However, in the case of an AB heterozygote, both RE1-RE2 and RE1-RE3 fragments will bind to the spot, resulting in a double signal from both the RE2 and the RE3 label. Similarly, an RE1-RE3 allele can be converted into RE1-RE2 alleles that can be detected independently on the diversity array. Allelic variation that results in RE1-RE2 conversion into another RE1-RE2 fragment, or RE1-RE3 conversion into another RE1-RE3 fragment, can not be scored.

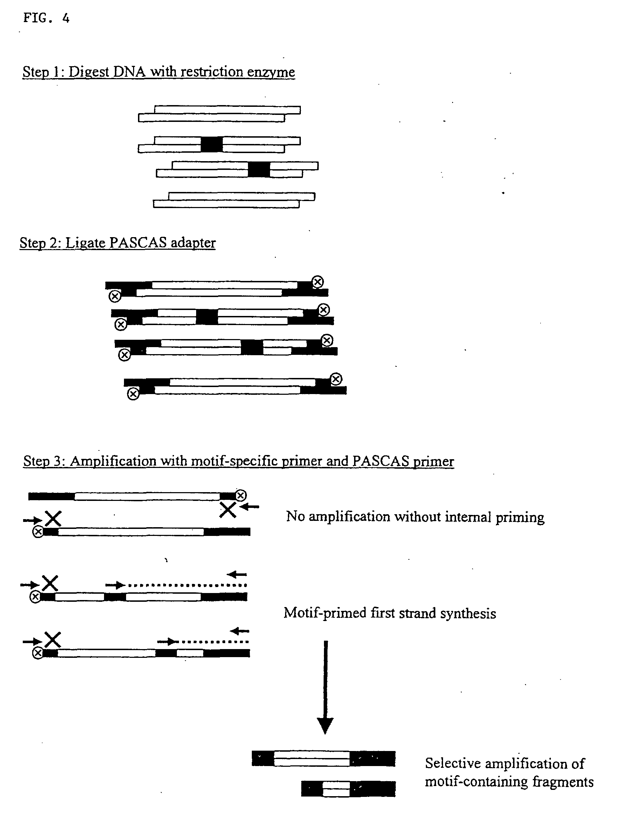

Figure 4: PASCAS adapter with motif-directed primer for generation of diversity panel. In the first step, genomic DNA is digested with a restriction enzyme or a combination of restriction enzymes. Then PASCAS adapters are ligated to all fragment ends. In step 3, PCR is performed with a PASCAS primer and a motif-specific primer that can bind to multiple targets in the genome. These can be for example members of gene families, promoter elements, retro-elements, or any other conserved sequence present in multiple copies in the genomic DNA. The primer can be specific or degenerated. The unique design of the PASCAS system inhibits amplification of any fragments from adapter primers alone. Only fragments that contain sequences to which the motif-specific primer can bind will be amplified, resulting a diversity panel that is highly enriched for fragment sequences flanking the targeted motif in the genomic DNA. The thus created motif-selected diversity panel can be used for specific fingerprinting techniques such as NBS-profiling and any variant of motif-directed profiling.

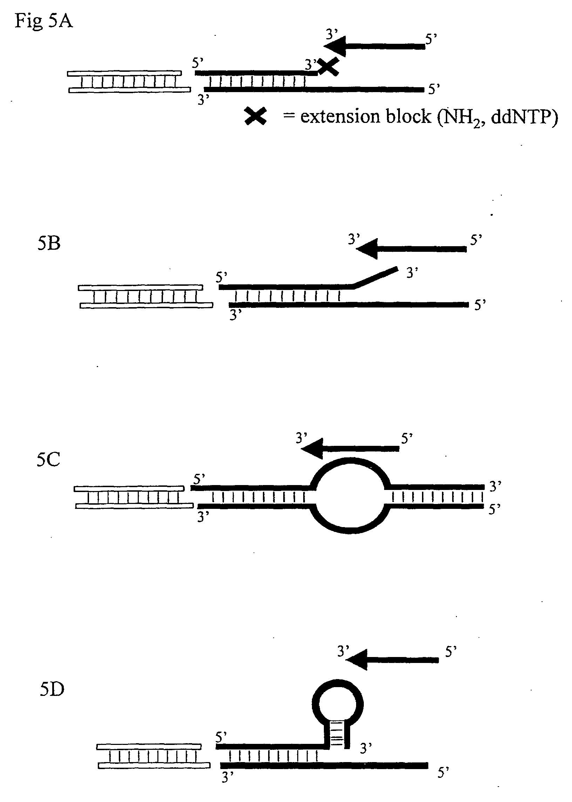

Figure 5 shows different examples of PASCAS adapters and accompanying primers. A more detailed description is given herein below.

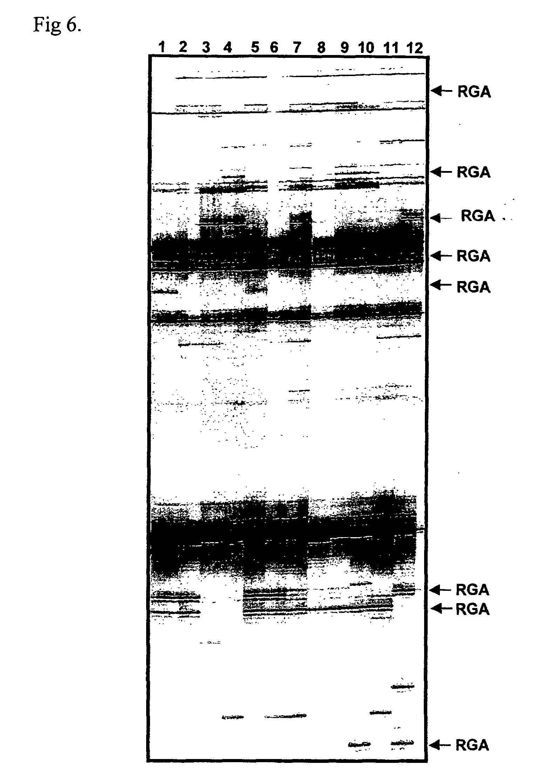

Figure 6 depicts a part of an NBS profile banding pattern in polyacrylamide gel for 13 potato varieties. For this NBS-profiling a PASCAS adapter was used, as explained in Example 1.

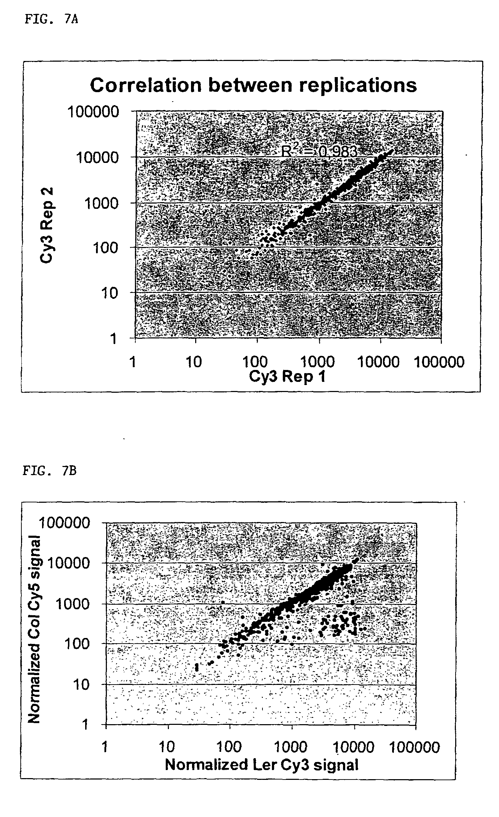

Figure 7 illustrates some results of the fingerprinting technology DArT, using the PASCAS system. From the *Arabidopsis thaliana* ecotype Landsberg erecta (Ler) a diversity panel was made by means of the PASCAS system. Amplicons were individualized by means of cloning, and spotted on a micro-array. The micro-array was hybridized with a Cy3-labeled diversity panel from genomic DNA from Ler, and a Cy5-labeled diversity panel from ecotype Colombia (Col). Figure 7A shows the relationship between the Cy3 signal in replication 1 and replication 2 on the micro-array. This graph illustrates the reproducibility within a slide. Col (Cy5) showed a similar reproducibility.
Figure 7B shows for the same micro-array as in Fig 7A the relationship between the Ler hybridization signal and the Col hybridization signal per spotted clone of Ler. A group of spots showed a reproducibly higher Ler signal than Col signal. These spots may contain Ler fragments that were present in the diversity panel from Ler but not in the diversity panel from Col. Therefore these spots are candidate markers for genetic differences between Col and Ler. This experiment is further explained in Example 2 herein below.

### Detailed description of the invention

### Definitions

In the description a number of terms are used herein. In order to provide a clear understanding, the following definitions are given:
- DNA fingerprinting. For reasons of consistency we use here the definition from the patent application for DArT (PCT international publication number WO 01/73119): A fingerprint comprises a distinct pattern of nucleic acid molecules that is a characteristic of the genotype of the organism from which the nucleic acids are prepared. A variety of techniques, such as restriction enzyme digestion and amplification, or other method can generate the patterns. Fingerprints can reveal sequence differences between nucleic acid samples that can be used to analyse and compare DNA from different species or different individuals of the same species.
- Diversity panel: A diversity panel refers to nucleic acid fragments prepared from organismal nucleic acids (e.g., genomic DNA or cDNA) by a method that can reveal polymorphisms or mutations (e.g., sequence differences) between samples (From WO 01/73119). In the present specification a diversity panel is also indicated as a reproducible selective set of DNA fragments from the DNA or cDNA of one or more organisms.
- Adapter: Short DNA molecule consisting of two (partly) complementary strands of DNA. The oligonucleotides comprising the adapter usually are synthetic. An adapter is designed in such a way that one end can be ligated to an end of a restriction fragment.
- PASCAS adapter. PASCAS is an acronym of Primer Annealing Site after Complementation of Adapter Strand. A PASCAS adapter is an adapter that is modified in such a way that the accompanying primer can not anneal efficiently to the adapter itself, but after complementing of one adapter strand the primer can anneal to this complement. One or more parts of one adapter strand are complementary to one or more parts of the other adapter strand. These complementary sequences allow formation of the double stranded structure, needed for ligation to a restriction fragment. However, one or more other parts of one strand have no complementary sequence in the other strand. Figure 1 shows an example. The sequence of one non-complemented part is identical or similar to the primer's sequence. When this adapter strand is complemented, a sequence is constructed that is complementary to the primer's sequence. This complementary sequence serves as an annealing site for the primer in such a way that it allows amplification during PCR (the primer can be extended in the direction of the ligation site). The adapter is modified in such a way that the synthesis of a complementary strand to which the adapter primer can anneal can not initiate from an available 3'-end in the adapter itself. Binding of the adapter primer therefore requires the synthesis of a complementary DNA strand that is initiated from another priming site outside of the modified adapter.

### Using the PASCAS system for reduction of the complexity of diversity panels

The general method involves the use of restriction endonucleases, also referred to as restriction enzymes. A restriction enzyme recognises a specific base sequence in a double-stranded DNA molecule, and cleaves the DNA duplex at or near this sequence to produce DNA fragments (restriction fragments). All restriction enzymes make two single-strand breaks but the ends have distinct properties. Some restriction enzymes make blunt ends where the bases at the end are paired. Other enzymes make cohesive, sticky ends in which one of the two strands protrudes. In case of sticky ends, adapters are used that have a single stranded protruding end that is complementary to the sticky end of the restriction fragment. The end of an adapter can then anneal to the end of a restriction fragment. Blunt end adapters can be ligated only to blunt fragment ends. The adapters can be attached to the ends of DNA fragments by covalent association, using a variety of techniques that are well known in the art, including ligase-mediated DNA ligation. For each type of sticky ends of restriction fragments specific adapters are used.

The DNA fragmentation step can be carried out with one or more restriction enzymes, followed by ligation of one or more adapters. Alternatively, the restriction and ligation can be performed in a single reaction. When restriction fragments that are cut by a single restriction enzyme are ligated to one another, the restriction site is recovered. In the single reaction situation with simultaneous restriction and ligation, these religated fragments will be cut again by the restriction enzyme. In order to prevent that in this mixture the restriction enzyme would also separate the adapter from the restriction fragment, it is preferred to design the adapter in such a way that the restriction site is not recovered after attachment of the adapter to the restriction fragment. The reaction is then driven to an effective ligation of the adapter(s) to restriction fragment ends.

In one embodiment two restriction enzymes are used, i.e. one rare cutter (RE1) and one frequent cutter (RE2). The rare cutter recognizes a specific sequence of 6 or more bases in the DNA. The frequent cutter recognizes a specific sequence of 4 to 6 bases. Cutting the DNA with these two enzymes results into three different types of restriction fragments, i.e. RE1-RE1 fragments, RE1-RE2 fragments, and RE2-RE2 fragments. RE1-RE1 fragments have been cut with the rare cutter RE1 at both sides. RE1-RE2 fragments have been cut by RE1 at one side and by RE2 at the other side, whereas RE2-RE2 fragments have been cut by RE2 at both ends. As RE1 sites are less abundant than RE2 sites, RE1 cuts less frequently than RE2, and the frequency of RE1-RE1 fragments is relatively low. The frequency of RE1-RE2 fragments is higher, and the frequency of RE2-RE2 fragments is the highest.

The complexity of this mixture of fragments can be reduced in numerous ways. This invention relates to the use of the PASCAS system for complexity reduction of diversity panels. In one preferred embodiment the complexity is reduced by amplification of only RE1-RE1 and RE1-RE2 fragments, without amplification of RE2-RE2 fragments. This is shown in Figure 1. In this embodiment a normal adapter is ligated to the RE1-ends, and a PASCAS adapter to the RE2-ends. An amplification primer for the RE1-adapter is added, and a PASCAS primer for the PASCAS-adapter at the RE2-end. In a first PCR thermocycle, the double stranded restriction fragments and the linked adapters are denatured. The primer for the RE1-adapter can subsequently anneal to the RE1-adapter, but at the RE2-ends with the PASCAS adapter, the PASCAS primer can not anneal. A DNA polymerase catalyses nucleotide extension of the RE1-primer using the adapter-ligated restriction fragment as template. In RE1-RE1 fragments, both strands are extended, and subsequent PCR thermocycles will result in exponential amplification of RE1-RE1 fragments.
In case of RE1-RE2 fragments, only the strand primed from the RE1 adapter is synthesized in the first PCR thermocycle. This newly synthesized strand now contains a binding site for the PASCAS primer at the RE2-end. Therefore, both primers will be extended in a second thermocycle, resulting in exponential amplification of RE1-RE2 fragments in subsequent thermocycles.
In case of RE2-RE2 fragments, the RE2-primer can not anneal to either of the RE2-adapter strands, so no primer extension takes place, and no annealing site for the RE2 adapter primer is made. Therefore RE2-RE2-fragments are not amplified in this embodiment. As a result the diversity panel consists of RE1-RE1 and RE1-RE2 fragments only.

The complexity of the diversity panel can be reduced further in numerous ways, such as using one or more extra restriction enzymes, without matching adapters. When an extra enzyme cuts an RE1-RE1 fragment or RE1-RE2 fragment, amplification of that fragment is prevented. Some other approaches, such as suppression PCR and the use of selective bases are mentioned above in 'Background of the invention'. These other complexity reduction methods can be combined with the invention.

### Structure and examples of modified adapters

PASCAS adapters can be designed in various ways. The typical property of the PASCAS system is that the primer is not able to anneal to an adapter strand itself but can anneal to the complement of an adapter strand. This can be achieved by designing the adapter in such a way that the one adapter strand is not completely complementary to the other adapter strand. Only a part is complementary to allow the formation of a stable double stranded structure. This double stranded structure should facilitate attachment of the double stranded adapter side to the restriction fragment. If the restriction fragment has a sticky end, the adapter should have a complementary sticky end to match the fragment end.
The PASCAS primer is identical or similar to the single stranded part of the PASCAS adapter. Consequently, this primer can not anneal to the adapter itself. It requires synthesis of the complementary strand of the single stranded part of the PASCAS adapter. Then the PASCAS primer can anneal to this complementary strand. This complementary strand can be synthesized if a primer is extended in the opposite direction. Once this first complementary strand is generated, the PASCAS primer can participate in a PCR reaction. The PASCAS adapter is modified in such a way that the synthesis of a complementary strand to which the adapter primer can anneal can not initiate from an available 3'-end of the adapter itself. Binding of the adapter primer therefore requires the synthesis of a complementary DNA strand that is initiated from another priming site outside of the modified adapter.

Examples of modified adapters are:
1. An adapter with a block at the 3' end of a short adapter strand. This is illustrated in Figure 5A. In this example the lower strand is longer then the upper strand. The upper strand anneals to the lower strand, thus creating a double stranded structure that can be attached to a restriction fragment. The lower strand has an extension of e.g. 15 to 30 nucleotides at the 5' end. The sequence of the accompanying amplification primer is at least partly identical to this part of the lower strand. The upper strand has a modification at the 3' end. This modification can be any modification that inhibits the enzymatic addition of nucleotides to this strand. An example of such a block is an amine group. This modification effectively blocks the extension of the upper strand with the lower strand as a template. If such a block would be absent, the upper PASCAS adapter strand would be extended by polymerase, using the lower PASCAS strand as template, thus creating an annealing site for the PASCAS primer, and losing the specific advantage of the PASCAS system. Because of the presence of the block, the creation of an adapter annealing site for the PASCAS primer is completely dependent on the creation of its complementary sequence from a primer outside of the PASCAS adapter.
   If the adapter were drawn at the left side of the restriction fragment, rather then at the right side, self-evidently the lower strand would have been shorter, and would have the block.
   The blocked adapter is described in the user manual of the universal GenomeWalker™ Kit of CLONTECH Laboratories (PT3042-1, page 29). In that context the blocked adapter is meant to facilitate the cloning of unknown flanking regions of DNA.
2. Adapters with non-complementary sequences of the two strands at the 3'-end of the short strand. An example of such an adapter is shown in Figure 5B. In this figure the short strand (upper) is complementary to the upper strand with the exception of at least two and possibly more nucleotides at the 3'-end. This mismatching of the 3'-end of the shorter strand effectively blocks extension of this strand. An annealing site for the accompanying PASCAL primer is thus only created when the complementary sequence of the longer adapter strand is synthesised from a primer outside of the PASCAS adapter.
3. A vectorette adapter, also referred to as bubble adapter. The vectorette adapter contains a central non-complementary mismatched region resulting in a bubble shape, as shown in Figure 5C. Again, the primer's sequence is similar to the sequence of the lower strand in the mismatch region.
4. Splinkerette adapter. In this type of adapter one strand has a hairpin structure at the distal end. This is shown in Figure 5D. The vectorette and splinkerette adapter are reviewed by Hui et al. (1998) in the context of methods for cloning unknown flanking DNA sequences.

Other modified adapters may be designed by those skilled in the art, based on two principles. The first principle is the presence of a double stranded DNA structure that allows attachment to the restriction fragment. The second principle is that one or both strands contain one or more parts that are not complementary to the other strand. The amplification primer for the modified adapter should be at least partly similar or equal to the sequence of such a single-stranded adapter sequence. Only after complementing of that single-stranded adapter sequence, the primer has an annealing site. The amplification primer should anneal to this complement in such a way that the 3' end of this primer points to the attached restriction fragment.

### Examples

### Example 1.

### Application of the PASCAS system for NBS profiling, using polyacrylamide electrophoresis

This example describes resistance gene (R-gene)- specific fingerprinting of potato genotypes by utilizing the PASCAS system for the generation of diversity panels. This is an R-gene specific variant of the motif-directed profiling approach. In this example, diversity panels are generated that display genetic variation in an important family of genes involved in disease resistance in plants. Disease resistance in plants often behaves according to a gene-for-gene relationship, where a single product in the pathogen (avirulence factor) is matched by a gene product from the plant host. The largest class of R-genes contains a conserved nucleotide binding domain (Nucleotide Binding Site, NBS), a Leucine-Rich Repeat (LRR) 3' of the NBS, and either a coiled-coil (CC) or a *Toll*-interleukin-like receptor (TIR) domain 5' of the NBS. A large number of NBS-LRR R-gene members are present in the plant genome (in *Arabidopsis thaliana* approximately 160, in rice more than 600).
For the R-gene targeted variant of Motif-directed-profiling (NBS profiling) degenerate primers were designed on highly conserved sequence motifs in the NBS domain of plant R-genes. These primers were designed to recognize a large number of putative NBS-containing R-genes while still retaining a high level of specificity for these R-genes. The R-gene specific primers were used together with the PASCAS system on DNA fragmented with a restriction enzyme. PCR resulted in an R-gene enriched diversity panel (as described in general in Figure 4). In this example, the genetic variation between diversity panels of different genotypes is visualized by radioactive labelling and subsequent polyacrylamide electrophoresis.
For NBS profiling of potato genotypes, plant material from tuber light sprouts of modern potato varieties was lyophilized. DNA was isolated essentially as described by Fulton *et a*/*.* (1995). For each genotype 400 ng of DNA was digested with *Rsa* I, a restriction enzyme leaving blunt fragment ends, in the appropriate reaction buffer for a total of 4 hours, with fresh enzyme added after 2 hours. The reaction was terminated by heat inactivation. A PASCAS adapter was ligated to the ends of the restriction fragments. This adapter was based on the one described in Fischer *et al*. (1995) but the 3'-end of the short strand was blocked for chain elongation by the presence of an amino group. The adapter's long strand sequence was 5'-ACTCGATTCTCAACCCGAAAGTATAGATCCCA-3'. The sequence of adapter's short strand was 5'-TGGGATCTATACTT-3', with the amine group at the 3' end. The 5'-end of this short strand was phosphorylated to facilitate ligation to blunt end fragments. For adapter ligation, a high concentrate ligase (5U/µl) was used for 16 hours at 20°C. The reaction was terminated by heat inactivation.
Amplification of NBS-specific fragments involved two steps. The first step was a linear (asymmetric) PCR in a PTC-200 thermocycler (MJ Research ™, Inc.) with a limited amount (1.5mM) of the NBS-specific primer (5'-YYTKRTHGTMITKGATGATGTITGG-3'), 10µM dNTPs, 0.4U HotStarTaq (Qiagen, Germany), 2.5µl HotStarTaq PCR buffer in a reaction volume of 25 µl. The program consisted of denaturation at 95 °C for 15 min, and then 30 cycles of 30 sec 95 °C, 1 min 40 sec 55 °C annealing and 2 min at 72 °C, and a final extension at 72 °C for 20 min. The asymmetric PCR was followed by an exponential PCR with NBS-primer and an PASCAS primer (5'-ACTCGATTCTCAACCCGAAAG-3') by adding to the linear PCR product: 15 pmol of each primer, 200 µM dNTPs, 0.4 U HotStarTaq, 2.5 µl HotStarTaq PCR buffer in an end volume of 50 µl. The cycling program and thermocycler were identical to that of the linear PCR.
Finally, the PCR products were labeled by primer extension, using the [γ -³³P]ATP end-labeled NBS-specific primer in a thermocycler for 10 cycles with HotStarTaq at conditions similar to the NBS-adapter primer PCR. The labeled PCR products were separated on a 6% polyacrylamide gel, and the individual fragments visualized by autoradiography.
Fig. 6 depicts part of the resulting autoradiogram. On the complete gel with 13 (randomly chosen) potato varieties ca. 65 bands are visible, of which about 50% are polymorphic.
In Motif-directed profiling approaches, the PASCAS system aims to prevent adapter to adapter amplification. The effectiveness of the PASCAS system in NBS profiling is illustrated by the fact that a profile in which the NBS primer was labeled was identical to a profile in which the adapter primer was labeled (not shown), indicating that the PASCAS system effectively inhibits the amplification of adapter-adapter fragments. The reduction of background fragments in the profile as a consequence of the use of the PASCAS system also allows easy recovery of bands and subsequent characterization by sequencing. The bands in the gel could be excised, reamplified and sequenced without the need for subcloning, which suggests that the bands indeed comprise of a single PCR fragment. In addition, all of the bands that were sequenced contained an NBS primer at one end and an adapter primer at the other end. More than 80% of the sequenced bands in this example were identified as putative R-genes. These included known R-genes as well as new R-gene analogs. Selectivity of NBS profiling for R-genes using the PASCAS system therefore appeared to be considerably higher than similar approaches that utilize other approaches for creating the diversity panels, like AFLP adapters and primers with selective nucleotides (Hayes and Saghai Maroof, 2000).

NBS profiling can be applied to resistance-related biodiversity studies, marker-assisted breeding for disease resistance, finding new resistance sources in available germplasm, and genetic mapping and cloning of R-genes.

### Example 2

Using the PASCAS system for DArT

Hereinbefore the DNA fingerprinting method DarT was referred to.

The main advantages of DArT are:
1) low cost per data point; 2) no sequencing is required; 3) no locus-specific or SNP-specific primers are required. This decreases developing time and costs considerably. 4) high throughput, and easy scaling-up by printing more spots on the array; 5) no gels or gel-reading required.

DArT is composed of two main steps. The first main step is the creation of a selective set of DNA fragments from the genomic DNA of one or more reference organisms. This is the diversity panel. These DNA fragments are individualized and placed onto an addressable array, such as a micro-array. The second main step is the creation of a similar diversity panel of DNA fragments from a test organism. These fragments are labeled and hybridized to the array. Non-hybridized fragments are washed off. The array is analyzed for the hybridized, labeled fragments from the test organism. The hybridization pattern reflects the genotype of the test organism. Different related test organisms can be genotyped in such a way.

This example describes how the PASCAS system was used for generation of diversity panels for the fingerprinting technology DArT. *Arabidopsis thaliana* was used as a model plant for proof of concept. The different steps were:
1. Making micro-arrays. The micro-arrays contained a diversity panel from the genomic DNA of the *A. thaliana* ecotype Landsberg erecta (Ler)
   1.1. Generation of a diversity panel of Ler. For this the PASCAS system was used;
   1.2. Individualization of amplicons from this diversity panel by cloning into a bacterial vector. This gives a library of individualized amplicons;
   1.3. Printing the individualized amplicons on a glass slide, resulting in a micro-array that contains genomic DNA fragments from Ler;
2. Using the micro-arrays for hybridization.
   2.1. Generation of targets for hybridization. Also here the PASCAS system was used. Targets from the genomic DNA of the *A. thaliana* ecotype Columbia (Col) served as a test sample and Ler targets as a reference. The Col targets and Ler targets were generated, using the same conditions as in step 1. The hybridization targets from Col were labeled with the fluorescent dye Cy5 and the hybridization targets from Ler with Cy3.
   2.2. Hybridization. The fluorescently labeled targets from Ler and Col were mixed and simultaneously hybridized to the micro-array.
   2.3. Scanning and data-analysis. The micro-arrays were scanned, and the images analyzed. Spots on the micro-array were selected that showed a significantly lower Col signal than Ler signal. Such a spot was likely to contain a fragment that hybridized with a target fragment from Ler, but the homologous target fragment was probably not amplified in Col. These polymorphic spots can be used as genetic markers.
3. Validation of the candidate markers.
   3.1. In order to validate these putative marker fragments, we took the Ler clones (see step 1) from which the polymorphic spots originated, and sequenced these clones. These Ler sequences were aligned to the published genome sequence of Col (Arabidopsis Genome Initiative, 2000). The Ler sequence and homologous Col sequence were compared, and checked for differences. In most cases, a recognition site for one of the restriction enzymes used for generation of the diversity panels was present in the Ler sequence, but absent in the corresponding Col sequence, due to single nucleotide polymorphisms (SNP's) or small deletions/insertions. These alignments revealed the cause of the absence of some Col targets, but presence of the corresponding Ler targets. This confirmed that the marker polymorphism was indeed based on genetic variation between Col and Ler.

The different steps are described in more detail below.

### Making micro-arrays

### Generation of a diversity panel of Ler, to be printed onto micro-arrays

Genomic DNA was extracted from flower buds from Ler and Col using the Retch procedure (Pereira and Aarts, 1998). Restriction and ligation were performed simultaneously to prevent fragment-to-fragment ligation. To about 500 ng of DNA, 50 µl of restriction ligation mixture was added and incubated for 2 hours at 37 °C. The restriction ligation mixture contained:
5 units of *Pst* I Restriction Enzyme 1 (RE1) and 5 units of *Eco* R I as second enzyme (RE2), 10 µl 5 x restriction-ligation buffer (10mM Tris.HAc, 10mM MgAc, 50mM KAc, 5mM DTT, 50 ng/µl BSA, pH 7.5), 10 mM ATP, 2 units T4 DNA ligase (invitrogen), 5 pmol of a non-modified adapter with a *Pst* I compatible end, and 50 pmol of a PASCAS adapter (Table 1) compatible with RE2 (*Eco* RI) fragment ends. The adapter strand sequences are given in Table 1. The NH₂ block at the 3'-end of the short *Eco* RI adapter strand inhibited extension of this oligo. To further reduce the complexity of the diversity panel, a third restriction enzyme (*Taq* I, 5 units) was added to the mixture, and incubated for 2 hours at 65°C. Only fragments that had at one side a *Pst* I adapter, and at the other end a *Pst* I or *Eco* R1 adapter could be amplified. Fragments that were cut by *Taq* I were not amplified.

A PCR was performed on this restriction-ligation mixture. The restriction-ligation mixture was diluted 20-fold with MQ. 12.5 µl diluted mix was used as a template in a 50 µl PCR reaction with 1.5 units of Taq polymerarse (Promega) and primers listed in Table 1. A touchdown PCR program was used on a MJ thermal cycler. The amplification mixture was incubated for 2 min at 94 °C, followed by 8 cycles of 94 °C for 10 sec, 65 °C for 30 sec, and 72 °C for 2 min, and 29 cycles of 94 °C for 10 sec, 56 °C annealing temperature for 30 sec, and 72 °C for 2 min. The annealing temperature was lowered during the first 8 cycles with 1 °C per cycle. The final extension was at 60 °C for 30 min. The PCR products were purified, using the Qiagen PCR purification kit.

### Generation of a library of individualized amplicons

A library of individualized amplicons from the Ler diversity panel was made by ligation into a vector, using the pGEM-T Easy vector system (Promega). The vectors were transformed into a heat-shock competent *E. coli* XL1-blue strain according to the manufacturer's protocol. Transformants were selected on LB medium containing ampicillin, X-gal and IPTG. Transformed white colonies were picked and transferred to 96-wells plates containing 70 µl LB freeze and incubated for 24 hours at 37 °C. One µl served as a template for a 100 µl PCR reaction containing 10 µl 10 X buffer, 10 µl M 13 Forward primer (10pmol/µl), 10 µl M 13 Reverse primer (10pmol/µl), 2 µl dNTPs (10mM), and 0.5 µl PromegaTaq (5U/µl). The PCR program started with 6 minutes denaturation at 95 °C, and continued with 34 cycles of 30 sec 95 °C, 30 sec 55 °C, and 2 min 72 °C.

### Printing on slides

The PCR products were purified with the QIAquick PCR purification kit (Qiagen). The purified PCR products were dried and resuspended in 10 µl 5 x SSC, and printed in duplo on corning GAPS II amino-silane coated slides, using a Cartesian Technologies spotter. The micro-arrays were dried overnight, and the fragments were linked to the slides using a UV crosslinker at 150 mJoules The slides were soaked twice in 0.2 % SDS for 2 min with gentle agitation and twice in MilliQ water for 2 min at room temperature. Then, the slides were put in boiling MilliQ water for 2 min for DNA denaturation. After drying at room temperature for 5 minutes, the slides were rinsed in 0.2 % SDS for 1 min and once in MilliQ water for 1 min at room temperature. The slides were submerged in boiling MilliQ water for 2 seconds, air dried, and stored in the dark at room temperature.

### Using the micro-arrays for hybridization

### Generation of targets for hybridization

The procedure for generation of Col and Ler targets for hybridization is identical to the one described above for making a diversity panel of Ler for printing on the slides. In short, DNA was cut with the restriction enzymes *Pst* I and *Eco* R1, and adapters were ligated. The adapter and primer sequences for the generation of the hybridization mixtures were different from the ones used for generation of fragments that were printed on the slides to prevent cross-hybridization of the primer sequences. The sequences are shown in Table 1. Fragments were cut with Taq1. Fragments with at the one side a *Pst* I adapter, and at the other side a *Eco* RI adapter or *Pst* I adapter without a *Taq* I site in between were amplified. The PCR products were purified and the DNA quantified using a spectrophotometer. The amplicons from Col were labeled with Cy5-dCTP and the amplicons from Ler (serving as a reference) with Cy3-dCTP- with the Bioprime DNA labeling System (Invitrogen). For this labeling 500 ng DNA per sample was used and 20 µl 2.5 x random priming mix in a total volume of 41 µl. The mixture was boiled for 5 minutes and put on ice. Then 5 µl 10x dNTP's (1.2mM each dNTP except dCTP at 0.6 mM), 3 µl Cy3-dCTP or Cy5-dCTP and 1 µl Klenow was added, giving a total volume of 50 µl. This mixture was incubated for 3 hours at 37 °C. The labeled sample was purified with a Qiagen column, and washed twice with PE buffer. The sample was eluted with 2 x 30 µl Qiagen elution buffer. The amounts of DNA and incorporated dye were measured by means of a spectrophotometer (Beckman Coulter DU® Serie 500 UV) according to the manufacturer's manual.

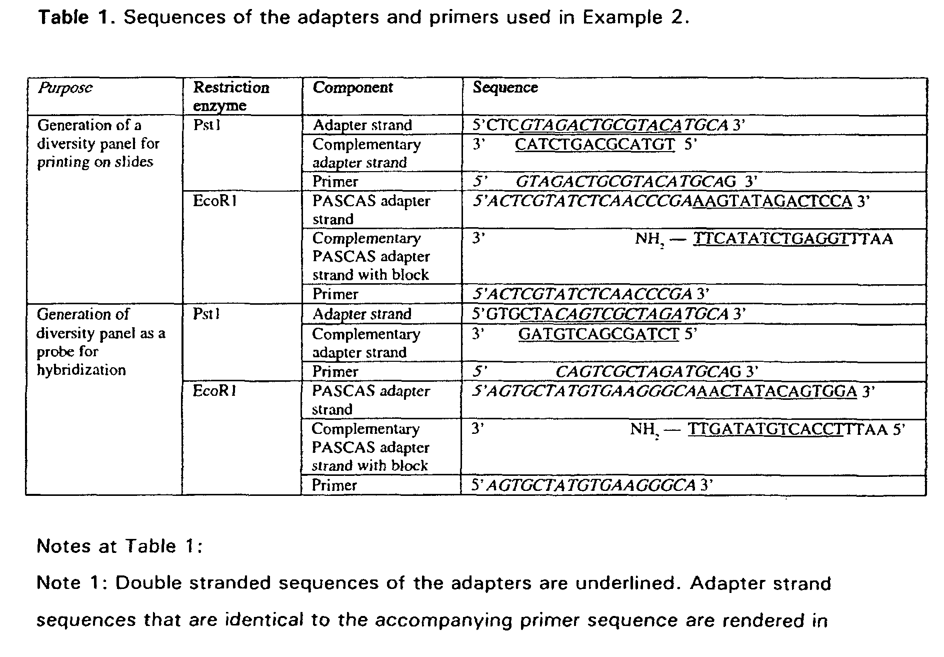

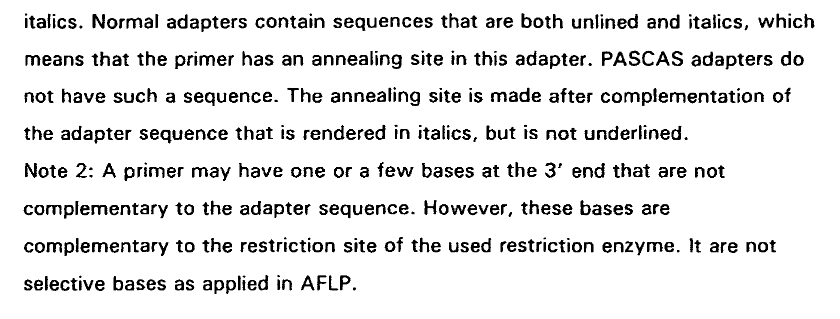

### Hybridization

The Cy3- and Cy5 labeled targets were pooled and the volume was reduced with a CentriconYM-30 column (Millipore) to 2-3 µl. To this mixture 40 µl of hybridization buffer was added. The hybridization buffer contained of 50 % formamide, 5 x Denhardt's reagent, 5 x SSC, 0.2 % SDS, and 0.1 mg/ml denatured (5 min 100 °C) herring sperm DNA. The sample was boiled for 2 min, cooled down to about 55 °C, and applied to the slide. A cover slip was put on the sample. The slide was incubated overnight in a humid hybridization chamber for 18 hours at 42 °C.
After hybridization the cover slip was removed, and the slide was rinsed with 1 x SSC and 0.1% SDS for 5 min in a black box with gentle stirring at room temperature. The washing was continued with 1x SSC for 5 min in a light-tight box, followed by 0.2 x SSC for 5 min, and 0.02 x SSC for 20 sec. The slide was dried by 1 min centrifugation at 1000 rmp, and stored in the dark at room temperature.

### Scanning an data-analysis

The slide was scanned with a Scanarray 3000 (General Scanning, Watertown, MA). A sequential scan was conducted for each of the two fluorescent dyes. The Cy3 and Cy5 intensities of each individual probe on the array were measured using the AIS software (Imaging research, Ontario, Canada).
Figure 7A shows the relationship of the hybridization signals of Cy3 in replication 1 and replication 2 on the same slide. A similar result was obtained for the Cy5 signals, This shows that the reproducibility within a slide was high. The reproducibility of the Cy3/Cy5 ratios between two separately performed experiments was also high (r = 0.97) starting from separately generated of diversity panels and hybridization on separate slides. Figure 7B shows the relationship between the Ler signal (Cy3) and the Col signal (Cy5) per clone for one slide, averaged among the two replications within the slide. Each point represents one Ler clone. For the majority of the spots the normalized signal for Ler approximately equaled the normalized signal for Col. This is the cluster of points around the 45° line. These points do not reveal genetic differences between Col and Ler. However, a group of spots showed a reproducibly higher signal for Ler than for Col. These spots are likely to represent fragments that were present in the hybridization target from Ler but absent in the hybridization target from Col, and can be used as genetic markers.

### Validation of candidate markers

The Ler clones corresponding to the polymorphic spots were sequenced. The majority of the cloned fragments contained at one side the *Pst* I adapter, and at the other side the *Eco* R1 adapter. No *Eco* R1 - *Eco* R1 fragments were found, indicating that the block at the *Eco* R1 PASCAS adapter was effective. A small number of fragments were *Pst* I *- Pst* I fragments, as the *Pst* I adapter was not a PASCAS adapter. The frequency of *Eco* RI sites is higher than the frequency of *Pst* I sites. As expected, the Ler fragments did not contain a Taq I site.

The sequences of the candidate polymorphic Ler fragments were compared to the published Col DNA sequence, using the BLAST program (Altschul *et al*, 1997. Nucleic Acids Res. 25, 3389-3402). The homologous Col sequences were checked for the presence of *Pst* I and *Eco* RI sites at the same locations as in the Ler sequences. Moreover it was checked whether a *Taq* I site was present in the homologous Col sequences. For 64 out of 71 polymorphic fragments the differential hybridization between Ler and Col could be explained by the absence of a *Pst* I or an *Eco* R1 site due to nucleotide substitutions or insertions/deletions, or the presence of a *Taq* I site in the Col sequence. For the remaining 7 clones we could not find a relevant difference between Col and Ler. This may be due to differential methylation between Col and Ler, as *Pst* I is a methylation-sensitive restriction enzyme.

It is concluded that the polymorphic spots in the *A. thaliana* diversity array generated with the aid of the PASCAS system were based on genetic variation, and as such are valid molecular markers.

### References

Altschul, S.F., Madden, T.L., Schäffer, A.A., Zhang, J., Zhang, Z., Miller, W., Lipman, D.J. (1997) Gapped BLAST and PSI-BLAST: a new generation of protein database search programs. Nucleic Acids Res. 25: 3389-3402.

Arabidopsis Genome Initiative, (2000). Analysis of the genome sequence of the flowering plant Arabidopsis thaliana. Nature 408 (6814): 796-815.

Fischer A., Saedler H., Theissen G. (1995). Restriction fragment length polymorphism-coupled domain-directed differential display: A highly efficient technique for expression analysis of multigene families. Proc. Natl. Acad. Sci. USA 92: 5331-5335.

Fulton T.M., Chunwongse J., Tanksley S.D. (1995) Microprep protocol for extraction of DNA from tomato and other herbaceous plants. Plant Mol. Biol. Rep. 13: 207-209.

Hayes, A.J., Saghai Maroof, M.A. (2000) Targeted resistance gene mapping in soy bean using modified AFLPs. Theor. Appl. Genet. 100: 1279-1283.

Hornstra IK, Yang TP (1993) In vivo footprinting and genomic sequencing by ligation-mediated PCR. Anal. Biochem. 213: 179-193.

Hui E K -W, Wang P -C, Lo S J (1998) Strategies for cloning unknown cellular flanking DNA sequences from foreign integrants. Cell. Mol. Life Sci. 54: 1403-1411.

Jaccoud D., Peng K., Feinstein D., Kilian, A. (2001) Diversity arrays: a solid state technology for sequence information independent genotyping. Nucleic Acids Res 29, No. 4e25.

Pereira, A., Aarts, M.G.M. (1998) Transposon tagging with the *En-I* system. In Arabidopsis Protocols, J. Martinez-Zapater and J. Sallinas, eds. (Totowa, NJ: Humana Press), pp. 3329-338.

Vos P, Hogers R, Bleeker R, Reijans M, Van de Lee T, Hornes M, Frijters A, Pot J, Peleman J, Kuiper M, Zabeau M (1995) AFLP: a new technique for FNA fingerprinting. Nucleic Acids Res 23: 4407-4414.

Waugh, R., McLean, K., Flavell, A.J., Pearce, S.R., Kumar, A., Thomas, B.B.T., Powell, W. (1997) Genetic distribution of Bare-1-like retrotransposable elements in the barley genome revealed by sequence-specific amplification polymorphisms (S-SAP). Mol. Gen. Genet. 253: 687-694.

Williams J.G.K., Kubelik A.R., Livak K.J., Raflaski J.A., Tingey S.V. (1990) DNA polymorphism amplified by arbitrary primers are useful as genetic markers. Nucl. Acid Res. 10: 6531-6535.

## Claims

1. A method for preparing a reproducible selective set of DNA fragments from the DNA or cDNA of one or more organisms as a main step in a DNA fingerprinting method, comprising
a) cutting DNA with one or more restriction enzymes to obtain a set of double stranded DNA fragments,
b) ligating to the DNA fragments double stranded adapters that match the generated fragment ends, and
c) performing a polymerase chain reaction using primers which are specific for the adapters to obtain the reproducible selective set of DNA fragments,
**characterized in that** in step b) at least one modified adapter is used which is composed of two oligonucleotides that are only partly complementary, leaving at least one part of the adapter single stranded, in such a way that a complementary strand of said single stranded part of the adapter cannot be synthesized from an available 3'-end in the adapter itself, and in step c) the primer which is specific for the modified adapter, is identical or similar to the single stranded part of the adapter, such that only after complementation of the single stranded part of the adapter said primer can anneal to the complement, and in such a way that this primer can be extended in the direction of the ligation site.

2. A method according to claim 1, wherein the modified adapter is selected from the group consisting of blocked adapters, vectorette adapters and spinklerette adapters.

3. A method according to claim 1 or 2, wherein the single stranded part of the modified adapter has a length of at least 8 nucleotides.

4. A method according to claim 3, wherein the single stranded part of the modified adapter has a length of 15 - 30 nucleotides.

5. A method according to any preceding claim, wherein in stap a) the DNA is cut with two restriction enzymes, RE1 and RE2, and in step b) the adapter which is specific for RE1 is a normal adapter, and the adapter which is specific for RE2 is a modified adapter.

6. A method according to claim 5, wherein RE1 is a rare-cutting enzyme and RE2 is a frequent-cutting enzyme.

7. A method according to any of claims 1 - 4, wherein in step a) the DNA is cut with three restriction enzymes, RE1, RE2 and RE3, in step b) the adapter which is specific for RE1 is a normal adapter, and the adapters which are specific for RE2 and RE3, respectively, are modified adapters, and in step c) the primers which are specific for the RE2-adapter and RE3-adapter, respectively, are labeled with labels that can be detected separately.

8. A method according to claim 7, wherein RE1 is a rare-cutting enzyme, and RE2 and RE3 are frequent-cutting enzymes.

9. A method according to any of claims 1 - 4, wherein in step b) the adapter(s) is (are) a modified adapter(s), and in step c) in addition to the primer(s) which is (are) specific for the adapter(s) used, a motif-specific primer is used that can bind to multiple targets in the DNA.

10. A method according to any preceding claim, which comprises digestion with one or more additional restriction enzymes during step a) or after step b) without ligation of adapters to the restriction fragment ends made by said additional restriction enzyme(s).

11. A method according to any preceding claim, wherein the DNA fingerprinting method is Diversity Array Technology.
